(19) Europäisches Patentamt
European Patent Office
Office européen des brevets

(11) EP 4 748 830 A1

(12) EUROPEAN PATENT APPLICATION
published in accordance with Art. 153(4) EPC

(43) Date of publication:
27.05.2026 Bulletin 2026/22

(21) Application number: 24842047.3

(22) Date of filing: 21.05.2024

(51) International Patent Classification (IPC):
*C07C 213/00* (2006.01) *C07C 209/00* (2006.01)
*C07C 211/54* (2006.01) *C08K 5/18* (2006.01)
*C08L 7/00* (2006.01) *C08L 9/00* (2006.01)

(52) Cooperative Patent Classification (CPC):
**B60C 1/00; C07C 209/00; C07C 211/54; C07C 213/00; C08K 3/04; C08K 3/06; C08K 3/38; C08K 5/18; C08K 7/26; C08K 13/04; C08L 7/00; C08L 9/00; C08L 61/12;** Y02T 10/86

(86) International application number:
PCT/CN2024/094380

(87) International publication number:
WO 2025/016047 (23.01.2025 Gazette 2025/04)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 18.07.2023 CN 202310885309

(71) Applicant: **Sennics Co., Ltd.**
Shanghai, 200120 (CN)

(72) Inventors:
- GAO, Yang
Shanghai 200120 (CN)
- WANG, Hao
Shanghai 200120 (CN)
- GUO, Xiangyun
Shanghai 200120 (CN)

(74) Representative: **V.O.**
P.O. Box 87930
2508 DH Den Haag (NL)

# (54) RUBBER COMPOSITION CAPABLE OF PROTECTING AGAINST METAL ION AGING AND USE THEREOF

(57) Disclosed are a compound of formula A that can be used as an anti-aging agent, a rubber composition containing the compound of formula A and a use of the rubber composition. The rubber composition of the present invention is used as an entire or partial rubber matrix of a tire, especially as a tire belt rubber. The anti-aging agent of the present invention can provide good rubber-steel wire adhesion performance, improve the thermal oxidative aging resistance of the rubber composition, and especially improve the mechanical properties and adhesion properties after aging in the presence of metal ions, prolonging the service life of tires.

EP 4 748 830 A1

## Description

### TECHNICAL FIELD

The present invention belongs to the field of a rubber, in particular to a rubber composition capable of protecting against metal ion aging and use thereof.

### BACKGROUND

As one of the main components of an automobile, a tire plays an important role in bearing vehicle weight and transmitting traction and braking forces. With the continuous development of the tire industry, radial tires have been widely adopted, and using the steel cords have become a very common reinforcement method. Therefore, the adhesion performance between a rubber and a steel wire has become crucial. Research has found that cobalt salts can significantly promote the rubber-steel cord adhesion. It is generally believed that cobalt salts enhance the activity of sulfur, increase the formation rate of copper sulfide at the adhesion interface, which is beneficial for improving adhesion performance.

Although the addition of cobalt salts can enhance rubber-steel cord adhesion performance, it is not detrimental to the aging performance of a rubber composition.

An unsaturated rubber is prone to react with oxygen. Research indicates that the oxidation of an unsaturated rubber is performed via a free radical chain reaction mechanism and has the characteristic of autocatalytic, while metal ions such as copper, manganese, and cobalt can play a catalytic role for the oxidation reaction of a rubber. Metal ion catalysis can greatly increase the oxidation rate, accelerate the thermal oxidation of rubber, which is of great harm to a rubber.

In the existing technology, an antidegradant is mostly used to inhibit the catalytic effect of metal ion, thereby improving the thermal oxidative aging resistance and other properties of a rubber product. Antidegradant 6PPD is a main antidegradant widely used in the rubber industry, which can inhibit ozone aging and thermal oxidative aging of a rubber product, and slow down rubber degradation under static conditions. However, antidegradant 6PPD exhibits certain polluting properties, and its extensive use as a single type in tire formulations increases production costs, and it cannot fully meet the requirements for performance retention under some specific aging conditions during the actual use of tires, and is prone to cause issues such as blooming.

### SUMMARY OF THE INVENTION

To overcome the problems in the prior art, the present invention provides a rubber composition with excellent protection against metal ion aging and use thereof. The rubber composition of the present invention is used as an entire or partial rubber matrix of a tire, especially as a tire belt rubber. The antidegradant of the present invention can provide good rubber-steel wire adhesion performance, improve the thermal oxidative aging resistance and protection against metal ion of a rubber composition, and especially improve the mechanical performance and adhesion performance after aging in the presence of metal ions, prolonging the service life of tires.

Specifically, one aspect of the present invention provides a compound of formula A which can be used as an antidegradant:

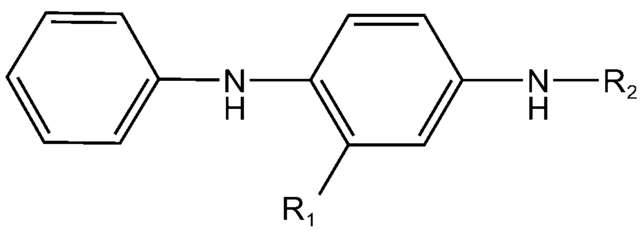

(A)

wherein, in formula A, $R_1$ is selected from C1-C8 alkyl, and $R_2$ is selected from C1-C8 alkyl.

In one or more embodiments, in formula A, $R_1$ is methyl, and $R_2$ is selected from C3-C6 alkyl.

In one or more embodiments, the compound of formula A is a compound of formula I, a compound of formula II, or a compound of formula III:

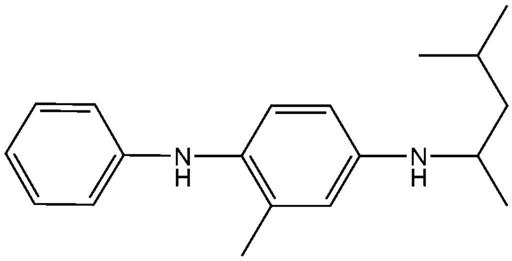

(I) ;

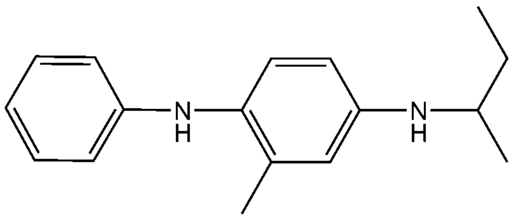

(II) ;

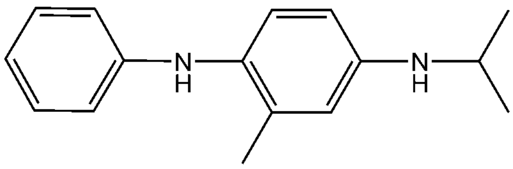

(III) .

In another aspect, the present invention provides a rubber composition, the raw materials of the rubber composition comprise 100 parts by mass of a diene elastomer and 0.5-5 parts by mass of a compound of formula A according to any embodiments of the present invention.

In one or more embodiments, the raw materials of the rubber composition comprise 100 parts by mass of a diene elastomer, 0.2-5 parts by mass of a cobalt salt, and 0.5-5 parts by mass of a compound of formula A according to any embodiments of the present invention.

In one or more embodiments, a diene elastomer is natural rubber.

In one or more embodiments, the raw materials of the rubber composition further comprise 2-10 parts by mass of sulfur.

In one or more embodiments, in the raw materials of the rubber composition, an amount of a compound of formula A is 1-3 parts by mass.

In one or more embodiments, the raw materials of the rubber composition further comprise 30-70 parts by mass of a reinforcing filler; the reinforcing filler preferably comprises carbon black and silica.

In one or more embodiments, the raw materials of the rubber composition further comprise 2-15 parts by mass of an activator; preferably, the activator is zinc oxide.

In one or more embodiments, the raw materials of the rubber composition further comprise 0.5-5 parts by mass of a tackifying resin.

In one or more embodiments, the raw materials of the rubber composition further comprise 1-10 parts by mass of a rubber adhesive.

In one or more embodiments, the raw materials of the rubber composition further comprise 0.5-5 parts by mass of an accelerator; preferably, the accelerator is N,N'-dicyclohexyl-2-benzothiazolesulfenamide.

In one or more embodiments, the raw materials of the rubber composition further comprise 0.02-0.5 parts by mass of a rubber scorch retarder.

In another aspect, the present invention provides a rubber product comprising the rubber composition according to any embodiments of the present invention; preferably, the rubber product is a tire belt.

In another aspect, the present invention provides a use of the compound of formula A according to any embodiments of the present invention in improving the mechanical performance and adhesion performance to steel wires after thermal oxidative aging in the presence of metal ion for a rubber composition or rubber product.

In one or more embodiments, the use comprises adding 0.5-5 parts by mass of the compound of formula A to the

raw materials of a rubber composition which comprises 100 parts by mass of a diene elastomer and 0.2-5 parts by mass of a cobalt salt.

## DETAILED DESCRIPTION OF THE INVENTION

In order to enable those skilled in the art to understand the characteristics and effects of the present invention, the terms and expressions mentioned in the description and claims are generally described and defined below. Unless otherwise specified, all technical and scientific terms used in the text have their usual meanings as understood by those skilled in the art regarding the present invention. In the event of conflict, the definitions in this specification shall prevail.

The theories or mechanisms described and disclosed herein, whether true or false, should not limit the scope of the invention in any way, that is, the invention may be implemented without being limited to any specific theory or mechanism.

In the present invention, "comprises", "includes", "contains" and similar terms encompass the meanings of "consisting essentially of" and "consisting of", for example, when it is disclosed herein that "A includes B and C", "A consists essentially of B and C" and "A consists of B and C" shall be deemed to have been disclosed herein.

In the present invention, all characteristics such as numerical values, amounts, contents, and concentrations defined in the form of numerical ranges or percentage ranges are for brevity and convenience only. Accordingly, descriptions of numerical ranges or percentage ranges shall be deemed to cover and specifically disclose all possible subranges and individual values within the ranges (including integers and fractions).

In the present invention, unless otherwise specified, percentage refers to mass percentage, and ratio refers to mass ratio.

In the present invention, when describing embodiments or examples, it should be understood that it is not intended to limit the present invention to these embodiments or examples. Conversely, all substitutes, modifications and equivalents of the methods and materials described in the present invention may be covered within the scope of the claims.

In the present invention, for the sake of conciseness, not all possible combinations of each technical feature in each embodiment or example are described. Thus, as long as there is no contradiction in the combination of these technical features, each technical feature in each embodiment or example may be arbitrarily combined, and all possible combinations should be considered to be within the scope of the specification.

The present invention has found that the compound of formula A can be used as a rubber antidegradant:

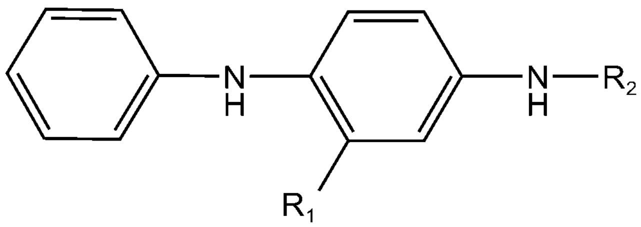

(A)

wherein, in formula A, $R_1$ is selected from C1-C8 alkyl, $R_2$ is selected from C1-C8 alkyl.

In the present invention, an alkyl group refers to a monovalent saturated group consisting of hydrogen atoms and carbon atoms. An alkyl group may be a linear or branched alkyl group. An alkyl group may contain 1 to 8 carbon atoms (C1-C8 alkyl). Examples of an alkyl group include, but are not limited to, methyl, ethyl, isopropyl, n-butyl, isobutyl, 1-methylpropyl, 1,3-dimethylbutyl, 1,4-dimethylpentyl, and octyl, etc.

In some embodiments, in the compound of formula A, $R_1$ is selected from C1-C4 alkyl, for example C1-C2 alkyl. In some embodiments, in the compound of formula A, $R_1$ is methyl.

In some embodiments, in the compound of formula A, $R_2$ is selected from C3-C6 alkyl. In some embodiments, in the compound of formula A, $R_2$ is selected from isopropyl, 1-methylpropyl, or 1,3-dimethylbutyl.

In some embodiments, in the compound of formula A, $R_1$ is selected from C1-C4 alkyl, for example C1-C2 alkyl; $R_2$ is selected from C1-C8 alkyl, for example C3-C6 alkyl.

In some embodiments, in the compound of formula A, $R_1$ is methyl, and $R_2$ is selected from C3-C6 alkyl.

In some embodiments, the compound of formula A is a compound of formula I, a compound of formula II, or a compound of formula III as shown below:

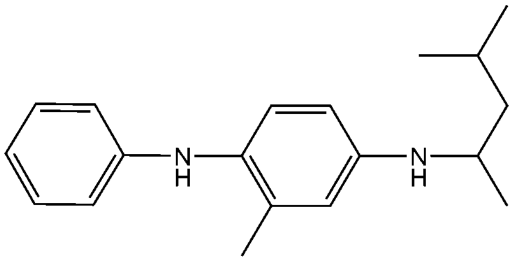

(I) ;

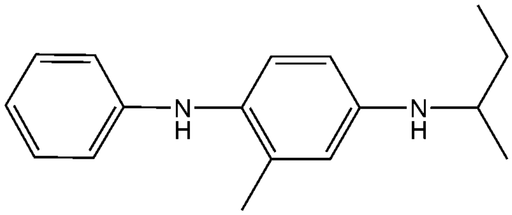

(II) ;

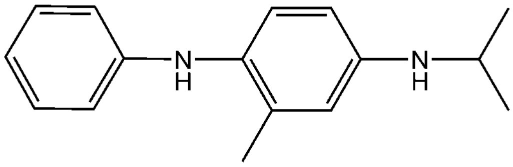

(III) .

The present invention has found that adding a compound of formula A (e.g., a compound of formula I, a compound of formula II, or a compound of formula III) to a rubber composition (e.g., a rubber composition for tires, particularly a rubber composition for tire belts) can provide excellent thermal oxidative aging resistance and good adhesion performance to steel wire for a rubber composition, especially thermal oxidative aging resistance in the presence of metal ion (e.g., a cobalt salt), significantly improving the mechanical properties and rubber-steel wire adhesion strength after thermal oxidative aging in the presence of metal ion for a rubber composition.

The present invention provides a method for preparing the compound of formula A, which comprises the following steps:

(1) performing a condensation reaction of aniline and a compound of formula B in the presence of a first catalyst to obtain a condensate comprising a compound of formula C and/or a compound of formula C', and then performing a reduction reaction of the condensate in the presence of $H_2$ and a second catalyst to obtain a compound of formula D;

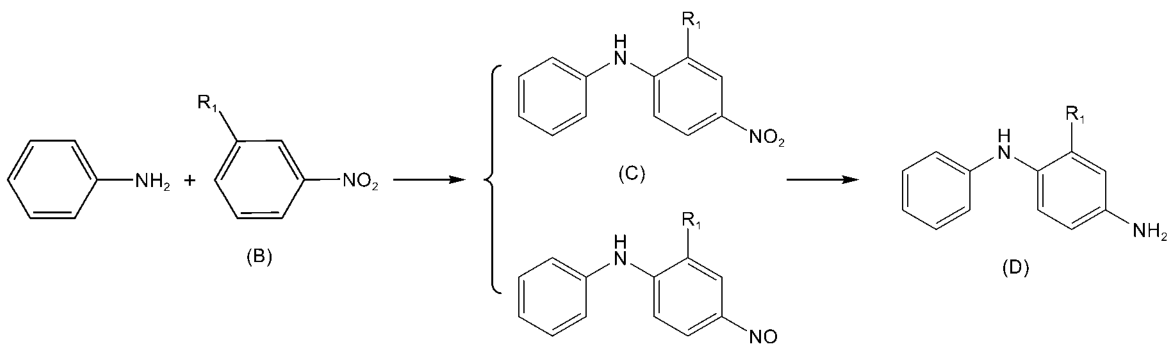

(B) (C) (D)

(C')

(2) performing a reductive alkylation reaction of the compound of formula D and a compound of formula E in the presence of $H_2$ and a third catalyst to obtain a compound of formula A;

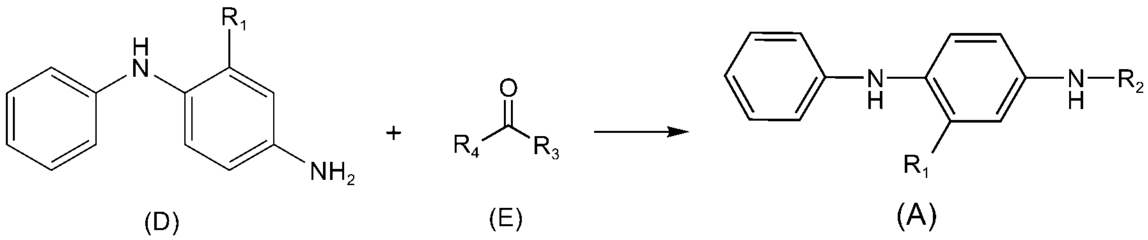

in Formula A, Formula B, Formula C, Formula C' and Formula D, each $R_1$ and $R_2$ are as defined in any embodiments of the present prevention. In Formula E, $R_3$ and $R_4$ are each independently selected from H and C1-C7 alkyl. Those skilled in the art can determine the corresponding $R_3$ and $R_4$ in Formula E based on $R_2$ in the compound of Formula A.

A first catalyst used in step (1) may be one or more selected from an alkali metal hydroxide, an alkali metal alkoxide, a quaternary ammonium base, and a combination of an alkali metal hydroxide and a halide of tetraalkylammonium. An alkali metal hydroxide suitable for the present invention includes sodium hydroxide, potassium hydroxide, lithium hydroxide, etc. An alkali metal alkoxide suitable for the present invention includes sodium methoxide, potassium methoxide, sodium ethoxide, potassium ethoxide, sodium tert-butoxide, potassium tert-butoxide, sodium tert-pentoxide, potassium tert-pentoxide, etc. A quaternary ammonium base is a compound having the general formula $(R_a)_4NOH$, wherein $R_a$ is four identical or different aliphatic hydrocarbon groups (such as alkyl) or aromatic hydrocarbon groups. In some embodiments, each $R_a$ in a quaternary ammonium base is an alkyl group, for example, each $R_a$ may be independently selected from methyl, ethyl, propyl, and butyl. Examples of a quaternary ammonium base suitable for the present invention include tetraalkylammonium hydroxides, for example, tetramethylammonium hydroxide, tetraethylammonium hydroxide, and tetrabutylammonium hydroxide. The halide of tetraalkylammonium has the general formula $(R_b)_4NX$, wherein $R_b$ is four identical or different aliphatic hydrocarbon groups (such as alkyl) or aromatic hydrocarbon groups, such as methyl, ethyl, propyl, butyl, etc., and X is a halogen atom, such as fluorine, chlorine, bromine, and iodine. The first catalyst may also be a combination of an alkali metal hydroxide and a halide of tetraalkylammonium. Examples of the combination of an alkali metal hydroxide and a halide of tetraalkylammonium include sodium hydroxide and tetrabutylammonium bromide. In some embodiments, the first catalyst is a quaternary ammonium base, such as tetraalkylammonium hydroxide. A molar ratio of a first catalyst to aniline may be 0.1:1 to 2:1, preferably 0.1:1 to 0.5:1, such as 0.2:1, 0.3:1, and 0.4:1. In step (1), aniline may firstly react with the first catalyst to form a salt, and then a compound of formula B is added dropwise for a condensation reaction.

In step (1), the condensate is obtained by the condensation reaction of aniline and the compound of formula B in the presence of the first catalyst, and the condensate may be one or two of a compound of formula C and a compound of formula C', and may also contain an azobenzene compound:

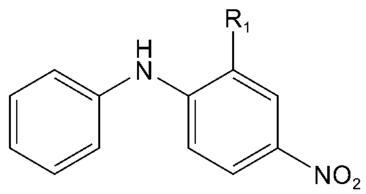

(C)

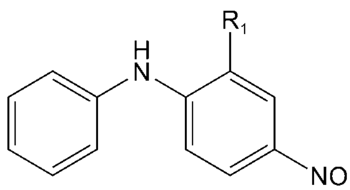

(C')

.

In step (1), a molar ratio of aniline to the compound of formula B may be 2:1~15:1, preferably 4:1~10:1, and more preferably 5:1~8:1, such as 6:1 or 7:1.

In step (1), the condensation reaction may be carried out at 40~90°C, preferably 65~85°C, for example, the reaction temperature may be 60°C, 70°C, 75°C, or 80°C. The condensation reaction needs to be carried out under vacuum with a pressure of -0.09~-0.99 MPa.

A second catalyst used in step (1) may be a porous metal catalyst or a supported metal catalyst. A porous metal catalyst is also known as a sponge metal catalyst. Porous metal catalysts suitable for the present invention include Raney nickel (also known as skeletal nickel), Raney cobalt, and Raney copper, etc. A supported metal catalyst comprises a metal that acts as center of catalytically activity and a support that is used for supporting the metal. The metal in the supported metal catalyst suitable for the present invention may be nickel, cobalt, copper, platinum, palladium, ruthenium, or rhodium, etc., and the support may be carbon, alumina, silica gel, or molecular sieves, etc. A carbon as a support may be activated carbon. In some embodiments, the second catalyst is a porous metal catalyst, such as Raney nickel. A molar ratio of a metal in the second catalyst to a condensate may be 0.0001:1~0.2:1.

In step (1), the condensate generated by the condensation reaction is subjected to a hydrogenation reduction reaction in the presence of a second catalyst to generate a compound of formula D. In step (1), the reduction reaction may be carried out at 40~120°C, preferably 60~90°C, for example, a reaction temperature may be 70°C, 75°C, or 80°C. A hydrogen pressure in the reduction reaction may be 0.5~5MPa, preferably 0.5~2.5MPa, such as 1MPa, 1.5MPa, 2MPa, or 2.5MPa.

In step (1), aniline may be used as a solvent, or a solvent such as toluene or xylene may also be used. After the completion of the reaction in step (1), the reaction solution is filtered to recover the second catalyst and the oil-water phase is separated to recover the first catalyst, and then the organic phase is distilled to remove the light components, thereby obtaining the compound of formula D.

A third catalyst used in step (2) may be the aforementioned supported metal catalyst, such as Pt/C. A molar ratio of the metal in the third catalyst to the compound of formula D may be 0.0001:1~0.2:1.

In step (2), the compound of formula D and the compound of formula E undergoes a hydrogenative reductive alkylation reaction in the presence of a third catalyst to generate the compound of formula A. After the reaction, the carbon atom of carbonyl in the compound of formula E is linked to the nitrogen atom of amino in the compound of formula D. Therefore, an appropriate compound of formula E may be selected for the reaction according to a $R_2$ group in the compound of formula A to be prepared. A molar ratio of the compound of formula E to the compound of formula D may be 1:1~15:1, such as 2:1, 3:1, 5:1, 8:1, 10:1, 11:1, or 14:1. A reaction temperature of step (2) may be 40~150°C, preferably 50~120°C, such as 50°C, 70°C, 80°C, 100°C, or 120°C. A hydrogen pressure in step (2) may be 0.5~5MPa, preferably 0.5~2.5 MPa, such as 1 MPa, 1.2 MPa, 1.5 MPa, 1.8 MPa, 2 MPa, or 2.5 MPa.

In step (2), the compound of formula E as a raw material for reaction may be used as a solvent. After the completion of the reaction in step (2), the reaction solution is filtered to recover the third catalyst, and distilled under reduced pressure to remove the light components, thereby obtaining the compound of formula A.

In the present invention, liquid chromatography (LC) or gas chromatography (GC) may be used to determine whether each step of the reaction reaches the endpoint, thereby determining the appropriate reaction time.

The present invention further provides a rubber composition which comprises the compound of formula A, such as a compound of formula I and/or a compound of formula II.

The raw materials of the rubber composition typically comprise a diene elastomer, a reinforcing filler, an antidegradant, and a crosslinking agent. In the present invention, a rubber composition comprises an unvulcanized rubber and a vulcanized rubber. A vulcanized rubber may be prepared by vulcanizing (curing) an unvulcanized rubber.

The raw materials of the rubber composition of the present invention comprise a diene elastomer and the compound of formula A as an antidegradant. An amount of the compound of formula A may be 0.5~5 parts by mass, based on 100 parts by mass of a diene elastomer. In the present invention, unless otherwise specified, the mass of a diene elastomer is taken as 100 parts by mass contained in the raw materials of the rubber composition as a benchmark to calculate the mass parts of other components in the raw materials of the rubber composition.

In the present invention, a diene elastomer refers to an elastomer whose monomer comprises a diene (such as butadiene, isoprene). A diene elastomer suitable for the present invention may be various diene elastomers known in the art, including but not limited to, one or more of natural rubber (NR), butadiene rubber (BR), isoprene rubber, styrene-butadiene rubber (SBR), chloroprene rubber (CR), nitrile rubber (NBR), an isoprene/butadiene copolymer, an isoprene/styrene copolymer, and an isoprene/butadiene/styrene copolymer. In some preferred embodiments, a diene elastomer comprises a natural rubber, or consists of a natural rubber.

An antidegradant contained in the rubber composition of the present invention comprises a compound of formula A (such as a compound of formula I and/or a compound of formula II). In some embodiments, the rubber composition of the present invention does not comprise other antidegradants except for the compound of formula A. In the raw materials of the rubber composition of the present invention, an amount of the compound of formula A may be 0.5-5 parts by mass, preferably 1-3 parts by mass, such as 1.2 parts by mass, 1.5 parts by mass, 1.8 parts by mass, 2 parts by mass, 2.2 parts by mass, or 2.5 parts by mass. In some embodiments, the raw materials of the rubber composition of the present invention comprise a compound of formula I and/or a compound of formula II, preferably comprising a compound of formula I. In some embodiments, an antidegradant in the rubber composition of the present invention is a compound of formula I, a compound of formula II, or a combination of a compound of formula I and a compound of formula II, preferably a compound of formula I. In the raw materials of the rubber composition of the present invention, an amount of a compound of formula I

when using a compound of formula I alone, an amount of a compound of formula II when using a compound of formula II alone, an amount of a compound of formula III when using a compound of formula III alone, or a total amount of two or three of a compound of formula I, a compound of formula II, and a compound of formula III when using two or three of a compound of formula I, a compound of formula II, and a compound of formula III at the same time, may be 0.5-5 parts by mass, preferably 1-3 parts by mass, such as 1.2 parts by mass, 1.5 parts by mass, 1.8 parts by mass, 2 parts by mass, 2.2 parts by mass, or 2.5 parts by mass.

The raw materials of the rubber composition of the present invention may comprise a reinforcing filler. In the raw materials of the rubber composition of the present invention, an amount of the reinforcing filler may be 30-70 parts by mass, such as 40 parts by mass, 45 parts by mass, 50 parts by mass, 55 parts by mass, or 60 parts by mass. A reinforcing filler suitable for the present invention may be a reinforcing filler conventionally used in a rubber composition, including but not limited to, one or more selected from the group consisting of carbon black, silica, titanium dioxide, calcium carbonate, magnesium carbonate, aluminum hydroxide, magnesium hydroxide, clay and talc. In some embodiments, a reinforcing filler comprises carbon black. Examples of carbon black include carbon black N330. In the rubber composition of the present invention, an amount of carbon black may be 25-55 parts by mass, such as 30 parts by mass, 35 parts by mass, 40 parts by mass, 43 parts by mass, 45 parts by mass, or 50 parts by mass. In some embodiments, a reinforcing filler comprises a silica. In the rubber composition of the present invention, an amount of a silica may be 5-20 parts by mass, such as 8 parts by mass, 10 parts by mass, 12 parts by mass, or 15 parts by mass. In some preferred embodiments, a reinforcing filler comprises carbon black and silica, or consists of carbon black and silica.

The raw materials of the rubber composition of the present invention comprise a crosslinking agent, such as sulfur. Sulfur is preferably an insoluble sulfur. In the raw materials of the rubber composition of the present invention, an amount of sulfur may be 2-10 parts by mass, such as 3 parts by mass, 4 parts by mass, 5 parts by mass, 6 parts by mass, 7 parts by mass, 8 parts by mass, or 9 parts by mass.

The raw materials of the rubber composition of the present invention may also comprise other components commonly used in a rubber composition, including but not limited to one or more selected from the group consisting of a softener, a protective wax, an activator, a cobalt salt, a tackifying resin, a rubber adhesive, an accelerator, a rubber scorch retarder, and the like.

A softener may be used to improve the processability of a rubber composition. Softener may include a petroleum-based softener such as naphthenic oil, aromatic oil, processing oil, lubricating oil, paraffin, liquid paraffin, petroleum bitumen, and petrolatum, etc., and may also include a fatty oil-based softener such as stearic acid, castor oil, linseed oil, rapeseed oil, coconut oil, waxes (such as beeswax, carnauba wax, and lanolin), tall oil, linoleic acid, palmitic acid, and lauric acid, etc. In the present invention, a softener is optionally added. When the raw materials of the rubber composition of the present invention comprise a softener, an amount of the softener may be 1-10 parts by mass, preferably 3-9 parts by mass, such as 4 parts by mass, 5 parts by mass, 6 parts by mass, 7 parts by mass, or 8 parts by mass.

A protective wax may migrate from the interior of a rubber to the surface to form a wax film, isolating the rubber surface from the external environment. In the present invention, a protective wax is optionally added. When the raw materials of the rubber composition of the present invention comprise a protective wax, an amount of the protective wax may be 1-5 parts by mass, such as 1.5 parts by mass, 2 parts by mass, 3 parts by mass, or 4 parts by mass.

An activator may play a role in accelerating the vulcanization rate, improving the thermal conductivity, wear resistance, and tear resistance of a rubber. The raw materials of the rubber composition of the present invention preferably comprise an activator. Examples of an activator includes ZnO. In the raw materials of the rubber composition of the present invention, an amount of activator may be 2-15 parts by mass, such as 5 parts by mass, 6 parts by mass, 7 parts by mass, 8 parts by mass, 9 parts by mass, 10 parts by mass, or 12 parts by mass. In some preferred embodiments, an activator includes ZnO, or an activator is ZnO. In some preferred embodiments, in the raw materials of the rubber composition of the present invention, an amount of ZnO is 2-15 parts by mass, such as 5 parts by mass, 6 parts by mass, 7 parts by mass, 8 parts by mass, 9 parts by mass, 10 parts by mass, or 12 parts by mass.

A cobalt salt may greatly help to promoting rubber-steel wire adhesion. When using as a rubber composition for tire belt, the raw materials of the rubber composition of the present invention preferably comprise a cobalt salt. Cobalt salts that can be used in the present invention include, but are not limited to, cobalt borate and cobalt neodecanoate. In the raw materials of the rubber composition of the present invention, an amount of a cobalt salt may be 0.2~5 parts by mass, such as 0.5 parts by mass, 0.8 parts by mass, 1 part by mass, 1.2 parts by mass, 1.5 parts by mass, 2 parts by mass, or 3 parts by mass.

A tackifying resin is used to enhance the adhesion of rubber to steel cord. When using as a rubber composition for tire belt, the raw material of the rubber composition of the present invention preferably comprises a tackifying resin. A usable tackifying resin includes a resorcinol donor, such as resorcinol-formaldehyde resin. In the raw material of the rubber composition of the present invention, an amount of a tackifying resin may be 0.5-5 parts by mass, such as 1 part by mass, 1.5 parts by mass, 2 parts by mass, 2.5 parts by mass, 3 parts by mass, and 4 parts by mass. In some embodiments, a tackifying resin comprises resorcinol-formaldehyde resin, or a tackifying resin is resorcinol-formaldehyde resin. Examples of usable tackifying resins include resorcinol-formaldehyde resin SL3022.

A rubber adhesive may be an adhesive containing a methylene donor (such as hexamethoxymethylmelamine), which is used in combination with a tackifying resin to fully adhere the rubber to a skeleton material such as a steel wire. When using as a rubber composition for tire belt, the raw materials of the rubber composition of the present invention preferably comprise a rubber adhesive. An available rubber adhesive includes a rubber adhesive with an active ingredient of hexamethoxymethylmelamine (HMMM), such as rubber adhesive RA. Rubber adhesive RA consists of HMMM and an inorganic carrier. Examples of rubber adhesive RA include rubber adhesive RA-65. The mass fraction of HMMM in rubber adhesive RA-65 is 65%. In the raw materials of the rubber composition of the present invention, an amount of rubber adhesive may be 1-10 parts by mass, such as 2 parts by mass, 3 parts by mass, 4 parts by mass, 5 parts by mass, 6 parts by mass, 7 parts by mass, or 8 parts by mass. In some embodiments, a rubber adhesive comprises rubber adhesive RA, or a rubber adhesive is rubber adhesive RA. In the raw materials of the rubber composition of the present invention, an amount of methylene donor (such as HMMM) may be 1-10 parts by mass, such as 2 parts by mass, 3 parts by mass, 4 parts by mass, 5 parts by mass, 6 parts by mass, 7 parts by mass, or 8 parts by mass.

A rubber scorch retarder is used to prevent the vulcanization of the rubber composition in the early stage of processing, and a rubber scorch retarder may be one or more selected from a nitroso compound (such as N-nitrosodiphenylamine), an organic acid (such as benzoic acid, phthalic anhydride), and a thioamide (such as N-cyclohexylthiophthalimide). Examples of rubber scorch retarders include rubber scorch retarder CTP (N-cyclohexylthiophthalimide). The raw materials of the rubber composition of the present invention preferably comprise a rubber scorch retarder. In the raw materials of the rubber composition of the present invention, an amount of a rubber scorch retarder may be 0.02-0.5 parts by mass, such as 0.05 parts by mass, 0.1 parts by mass, 0.2 parts by mass, 0.3 parts by mass, or 0.4 parts by mass. In some embodiments, a rubber scorch retarder comprises rubber scorch retarder CTP, or a rubber scorch retarder is rubber scorch retarder CTP.

An accelerator is usually a vulcanization accelerator, and may be one or more of a sulfonamide vulcanization accelerator, a thiazole vulcanization accelerator, a thiuram vulcanization accelerator, a thiourea vulcanization accelerator, a guanidine vulcanization accelerator, a dithiocarbamate vulcanization accelerator, a aldehyde amine vulcanization accelerator, a aldehyde ammonia vulcanization accelerator, an imidazoline vulcanization accelerator, and a xanthonic acid vulcanization accelerator. In the raw materials of the rubber composition of the present invention, an amount of an accelerator may be 0.5-5 parts by mass, such as 1 part by mass, 1.4 parts by mass, 1.5 parts by mass, 2 parts by mass, or 3 parts by mass. In some preferred embodiments, an accelerator is accelerator DZ (N,N'-dicyclohexyl-2-benzothiazolylsulfenamide).

In addition, if it is required, the rubber composition may further comprise a plasticizer such as DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate). An amount of a plasticizer may be a routine amount in the art.

When the rubber composition is used as a rubber composition for tire belt, the rubber composition of the present invention preferably comprises a bonding system consisting of a resorcinol donor (such as resorcinol-formaldehyde resin), a methylene donor (such as HMMM) and silica, which may effectively improve the adhesion of rubber to metal. An amount of a resorcinol donor, a methylene donor, and silica can be as described in any embodiments of the present invention.

In some preferred embodiments, the raw materials of the rubber composition of the present invention comprise: 100 parts by mass of a diene elastomer, 2-10 parts by mass of sulfur, 0.5-5 parts by mass of a compound of formula A, 25-55 parts by mass of carbon black, 5-20 parts by mass of silica, 2-15 parts by mass of ZnO, 0.2-5 parts by mass of a cobalt salt, 0.5-5 parts by mass of a tackifying resin (such as resorcinol-formaldehyde resin), 1-10 parts by mass of a rubber adhesive (such as rubber adhesive RA), 0.02-0.5 parts by mass of a rubber scorch retarder (such as rubber scorch retarder CTP), and 0.5-5 parts by mass of an accelerator (such as accelerator DZ), or consist of the above components.

An unvulcanized rubber of the present invention may be prepared by a conventional rubber mixing method, for example, it may be prepared by a two-stage mixing method: a first stage of thermomechanical mixing (e.g., using a internal mixer), mixing the raw materials of the rubber composition other than a crosslinking agent, a rubber adhesive, and an accelerator to obtain a mixture, and then kneading the mixture until reaching a maximum temperature between 110°C and 190°C to obtain a first stage rubber; a second stage of thermomechanical mixing (e.g., using an open mill), mixing the first stage rubber with a crosslinking agent, a rubber adhesive, and an accelerator to obtain a second stage rubber, i.e., an unvulcanized rubber.

An unvulcanized rubber of the present invention may be vulcanized by a conventional vulcanization method to obtain a vulcanized rubber. A vulcanization temperature is usually 130°C-200°C, such as 140-160°C or 150±5°C; a vulcanization time depends on a vulcanization temperature, vulcanization system, and vulcanization kinetics, and is usually 15-60 minutes, such as 20-40 minutes or 30±5 minutes.

Compared to a rubber composition using antidegradant 6PPD, the rubber composition of the present invention which use the compound of formula A (such as one or more of a compound of formula I, a compound of formula II, and a compound of formula III) as an antidegradant, is used in a rubber product such as a tire, especially a tire belt, so that the

rubber product has better mechanical properties and rubber-steel wire adhesion performance after thermal-oxidative aging in the presence of metal ion (such as cobalt ion). Therefore, the present invention also provides a rubber product which comprises the rubber composition described in the present invention. A rubber product may be a tire, a rubber shoe, a sealing strip, an acoustic panel, and a crash pad, etc. Preferably, a rubber product is a tire, such as a tire belt.

The present invention further provides a use of the compound of Formula A (e.g., one or more of a compound of formula I, a compound of formula II, and a compound of formula III) in improving the mechanical performance and rubber-steel wire adhesion performance after thermo-oxidative aging in the presence of metal ion (e.g., cobalt ion) for a rubber composition or rubber product, and also provides a method for improving the mechanical performance and rubber-steel wire adhesion performance after thermo-oxidative aging in the presence of metal ion (e.g., cobalt ion) for a rubber composition or rubber product. Preferably, the use or method of the present invention comprises: adding the compound of formula A (e.g., one or more of a compound of formula I, a compound of formula II, and a compound of formula III) to the raw materials of a rubber composition. In the use or method of the present invention, an amount of the compound of formula A (e.g., one or more of a compound of formula I, a compound of formula II, and a compound of formula III) and the composition of the raw materials of the rubber composition are preferably as described in any embodiments of the present invention.

The present invention is described in the following examples. These examples are merely illustrative and are not intended to limit the scope of the present invention. The methods, reagents, and materials used in the examples, unless otherwise stated, are conventional methods, reagents and materials in the art. The raw materials used in the examples are commercially available.

The sources and specifications of the raw materials used in the examples are as follows:

Natural rubber (NR): Xishuangbanna Sinochem Rubber Co., Ltd., SCR5;
Carbon black: Shanghai Cabot Carbon Black Co., Ltd., N330;
Silica: Shanghai Cain Chemical Co., Ltd., VN3;
Zinc oxide: Yonghua Chemical Technology (Jiangsu) Co., Ltd.;
Cobalt borate: Zhejiang Jinmao Rubber Auxiliaries Products Co., Ltd., compliant with industry standard HG/T 4072-2008;
Antidegradant 6PPD: Sennics Co., Ltd.;
Resorcinol-formaldehyde resin: SI Group Co., Ltd., SL3022;
Rubber adhesive RA-65: Shandong Ruiqi Chemical Co., Ltd.;
Rubber scorch retarder CTP: Shandong Yanggu Huatai Chemical Co., Ltd.;
Insoluble sulfur: Sennics Co., Ltd., HD OT20;
Accelerator DZ: Willing New Materials Technology Co., Ltd.

Preparation Example 1

(1) Synthesis of Compound IV

139.7g (1.5 mol) of aniline and 91g (0.25 mol) of 25% aqueous solution of tetramethylammonium hydroxide (TMAOH) are added into a 500mL four-mouth flask with stirring and the temperature is raised to 40~55°C. TMAOH and aniline are formed into salts by distillation and dehydration under reduced pressure. During the process, the color of the reaction liquid gradually changes from yellow to dark-red. The temperature is gradually raised to 72°C. When the amount of fraction is about 46 mL, distillation under reduced pressure(-0.098MPa) at 72°C and dropwise addition of 34.3g (0.25mol) 3-methyl nitrobenzene are performed at the same time, and the dropwise addition time is about 3 hrs. After the dropwise addition, the temperature is kept for 1 hr. The reaction is monitored by LC until 3-methyl nitrobenzene is completely reacted. A condensate liquid is obtained.

The above condensate liquid is transferred to a 500mL stainless steel reactor, to which 50g of deionized water and 40g of skeleton nickel catalyst are added. The atmosphere is replaced with hydrogen for three times, the temperature is raised to 75°C and hydrogen is introduced to raise the pressure to be 1.5 MPa for a hydro-reduction reaction. The reaction is monitored by LC until nitro-compounds and nitrosocompounds are completely reduced. Then the reaction liquid is filtered and separated to obtain an organic phase, and the organic phase is washed with water and distilled under reduced pressure (-0.1MPa, 160°C) to remove aniline and by-products of light components. Finally, 42.6g of a reduction product (Compound IV) is obtained through rectification, and it is cooled and solidified into a yellow solid. The yield of Compound IV is about 86% and its content is >99% by GC detection.

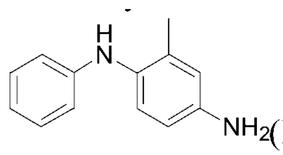

(IV)

LC-MS (m/z): 198.22 (M-H+).

1H NMR (400 MHz, CDCl3) δ 7.22 - 7.15 (m, 2H), 7.03 (d, J = 8.3 Hz, 1H), 6.79 - 6.74 (m, 1H), 6.68 (dt, J = 8.8, 1.7 Hz, 2H), 6.61 (d, J = 2.6 Hz, 1H), 6.54 (dd, J = 8.3, 2.7 Hz, 1H), 5.15 (s, 1H), 3.56 (s, 2H), 2.17 (s, 3H).

(2) Synthesis of Compound I

46g (0.23mol) of Compound IV, 70g (0.70mol) of 4-methyl-2-pentanone, and 0.8g of Pt/C catalyst are put into a reactor. The atmosphere is replaced with hydrogen for three times. The temperature is raised to 80°C, and hydrogen is introduced to raise the pressure to be 1.8 MPa for a reaction. The reaction is monitored by GC. When the content of Compound IV is <0.1% by GC detection, the reaction is stopped. The temperature is cooled down, the catalyst is removed by filtration, and the light components are removed by distillation under a pressure of -0.1MPa at 180°C to obtain 60.4g of Compound I (yield is about 93%), and its content is >95.8% by GC detection. After cooling and solidification, it is a red liquid.

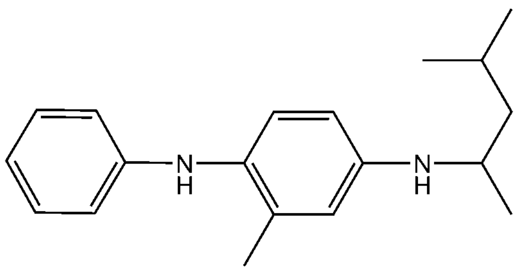

(I)

Molecular formula: $C_{19}H_{26}N_2$
LC-MS (m/z): 282.40 (M-H+).

Preparation Example 2

(1) Synthesis of Compound IV

The synthesis of Compound IV is the same as Preparation Example 1.

(2) Synthesis of Compound II

40 g (0.2 mol) of Compound IV, 151.4 g (2.1 mol) of 2-butanone, and 0.5 g of Pt/C catalyst are added into a reactor. The atmosphere is replaced with hydrogen for three times. The temperature is raised to 80°C, and hydrogen is introduced to raise the pressure to be 1.5 MPa for a reaction. The reaction is monitored by GC, when the content of Compound IV is <0.1%, the reaction is stopped. The temperature is cooled down. The catalyst is removed by filtration, and light components are removed by distillation under a pressure of -0.1 MPa at 180°C to obtain 50.9 g of Compound II (yield is about 99%), and its content is >99% by GC detection. After cooling and solidification, it is a light red solid.

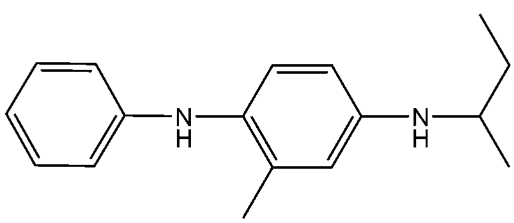

(II)

Molecular formula: $C_{17}H_{22}N_2$
LC-MS(m/z): 254.35 (M-H+).
$^1$H NMR (400 MHz, $CDCl_3$) δ 7.26 - 7.18 (m, 2H), 7.01 (d, *J* = 8.4 Hz, 1H),6.73 - 6.68 (m, 1H), 6.65 (m, 2H), 6.47 (d, *J* = 2.7 Hz, 1H), 6.43(dd, *J* = 8.4, 2.7 Hz, 1H), 4.98 (s, 1H), 3.68 - 3.42 (m, 1H), 3.30 (br.s, 1H), 2.20(s, 3H), 1.52-1.45 (m, 2H), 1.21 (d, *J* = 6.3 Hz, 3H), 0.97 (t, *J* = 7.4 Hz, 3H).

Preparation Example 3

(1) Synthesis of Compound IV

The synthesis of Compound IV is the same as Preparation Example 1.

(2) Synthesis of Compound III

40 g (0.2 mol) of Compound IV, 162.4 g (2.8 mol) of acetone, and 0.5 g of Pt/C catalyst are added into a reactor. The atmosphere is replaced with hydrogen for three times. The temperature is raised to 70°C, and hydrogen is introduced to raise the pressure to be 1.5 MPa for a reaction. The reaction is monitored by GC, when the content of Compound IV is <0.1%, the reaction is stopped. The temperature is cooled down. The catalyst is removed by filtration, and light components are removed by distillation under a pressure of -0.1 MPa at 180°C to obtain 48.3 g of Compound III (yield is about 99.4%), with a content of >98% by GC detection. After cooling and solidification, it is a light pink solid.

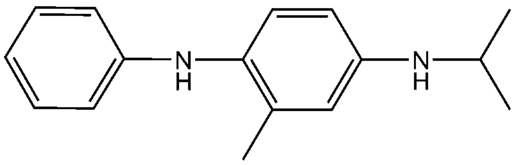

(III)

Molecular formula: $C_{16}H_{20}N_2$
LC-MS (m/z): 240.35 (M-H+).
$^1$H NMR (400 MHz, $CDCl_3$) δ 7.20 - 7.11 (m, 2H), 7.03 (d, *J* = 8.4 Hz, 1H), 6.77 - 6.70 (m, 1H), 6.65 (dt, *J* = 8.8, 1.7 Hz, 2H), 6.50 (d, *J* = 2.7 Hz, 1H), 6.44 (dd, *J* = 8.4, 2.7 Hz, 1H), 5.13 (s, 1H), 3.71 - 3.43 (m, 1H), 3.34 (br s, 1H), 2.17 (s, 3H), 1.23 (d, *J* = 6.3 Hz, 6H).

Examples 1-6 and Comparative Examples 1-4

According to the formulations shown in Table 1, the rubber composition of Examples 1-6 and Comparative Examples 1-4 are prepared using the following steps:
(1) A first-stage mixing is carried out in an internal mixer. The initial temperature of the internal mixer is 70°C, and the rotor speed is 60 r·min$^{-1}$. Natural rubber is added into the internal mixer, followed by the addition of carbon black N330, silica, zinc oxide, cobalt borate, antidegradant (a compound of Formula I, a compound of Formula II, or antidegradant 6PPD), resorcinol-formaldehyde resin SL3022, and rubber scorch retarder CTP. The mixture is kneaded until reaching a temperature of 150°C and then discharged to obtain a first-stage rubber.
(2) A second-stage mixing is carried out on an open mill. The first-stage rubber, rubber adhesive RA-65, insoluble sulfur, and accelerator DZ are added into the open mill. After the mixture was sheeted off after 5 passes, a second-stage rubber is obtained.
(3) The second-stage rubber is vulcanized at 151°C for 30 minutes to obtain a vulcanized rubber.

Table 1: Formulations of the rubber composition of Examples 1-6 and Comparative Examples 1-4 (unit: parts by mass)

| | Ex.1 | Ex.2 | Ex.3 | Ex.4 | Ex.5 | Ex.6 | Com.Ex.1 | Com.Ex.2 | Com.Ex.3 | Com.Ex.4 |
|---|---|---|---|---|---|---|---|---|---|---|
| Natural rubber | 100 | 100 | 100 | 100 | 100 | 100 | 100 | 100 | 100 | 100 |
| Carbon black N330 | 43 | 43 | 43 | 43 | 43 | 43 | 43 | 43 | 43 | 43 |
| Silica | 10 | 10 | 10 | 10 | 10 | 10 | 10 | 10 | 10 | 10 |

(continued)

| | Ex.1 | Ex.2 | Ex.3 | Ex.4 | Ex.5 | Ex.6 | Com.Ex.1 | Com.Ex.2 | Com.Ex.3 | Com.Ex.4 |
|---|---|---|---|---|---|---|---|---|---|---|
| Zinc oxide | 8 | 8 | 8 | 8 | 8 | 8 | 8 | 8 | 8 | 8 |
| Cobalt borate | 1 | 1 | 1 | 1 | 1 | 1 | 1 | 0 | 1 | 1 |
| Compound I | 2 | 1.5 | 0 | 0 | 0 | 0 | 0 | 0 | 0 | 0 |
| Compound II | 0 | 0 | 2 | 1.5 | 0 | 0 | 0 | 0 | 0 | 0 |
| Compound III | 0 | 0 | 0 | 0 | 2 | 1.5 | 0 | 0 | 0 | 0 |
| Antidegradant 6PPD | 0 | 0 | 0 | 0 | 0 | 0 | 0 | 0 | 2 | 1.5 |
| Resorcinol-formaldehyde resin SL3022 | 2 | 2 | 2 | 2 | 2 | 2 | 2 | 2 | 2 | 2 |
| Insoluble sulfur | 5 | 5 | 5 | 5 | 5 | 5 | 5 | 5 | 5 | 5 |
| Rubber adhesive RA-65 | 5 | 5 | 5 | 5 | 5 | 5 | 5 | 5 | 5 | 5 |
| Accelerator DZ | 1.4 | 1.4 | 1.4 | 1.4 | 1.4 | 1.4 | 1.4 | 1.4 | 1.4 | 1.4 |
| Rubber scorch retarder CTP | 0.1 | 0.1 | 0.1 | 0.1 | 0.1 | 0.1 | 0.1 | 0.1 | 0.1 | 0.1 |

Test Example 1: Mechanical Properties Before and After Thermal Oxidative Aging

A tensile strength and an elongation at break before thermal oxidative aging, and a retention rate of tensile strength and a retention rate of elongation at break after thermal oxidative aging, of the vulcanized rubbers are tested according to the following standards:

Tensile strength and elongation at break: GB/T 528-2009, Determination of tensile stress-strain properties of a vulcanized rubber or thermoplastic rubber.
Thermal oxidative aging test: GB/T 3512-2014, Vulcanized or thermoplastic rubber, Hot-air-accelerated aging and heat resistance test, Aging condition of 100°C × 48h.

Retention rate of tensile strength after aging = (Tensile strength after aging / Tensile strength before aging) × 100%

Retention rate of elongation at break after aging = (Elongation at break after aging / Elongation at break before aging) × 100%

The mechanical properties before and after aging for the rubber composition of Examples 1-6 and Comparative Examples 1-4 are shown in Table 2.

Table 2: Mechanical properties before and after aging for rubber composition of Examples 1-6 and Comparative Examples 1-4

| | Ex.1 | Ex.2 | Ex.3 | Ex.4 | Ex.5 | Ex.6 | Com.Ex.1 | Com.Ex.2 | Com.Ex.3 | Com.Ex.4 |
|---|---|---|---|---|---|---|---|---|---|---|
| Tensile strength before aging/MPa | 24.5 | 24.3 | 24.4 | 24.2 | 24.0 | 23.9 | 24.2 | 24.4 | 24.3 | 24.2 |
| Elongation at break before aging/% | 437 | 431 | 435 | 432 | 433 | 430 | 426 | 450 | 438 | 429 |

(continued)

| | Ex.1 | Ex.2 | Ex.3 | Ex.4 | Ex.5 | Ex.6 | Com.Ex.1 | Com.Ex.2 | Com.Ex.3 | Com.Ex.4 |
|---|---|---|---|---|---|---|---|---|---|---|
| Retention rate of tensile strength after aging under 100°C×48h /% | 69 | 68.2 | 65.8 | 65.1 | 63.7 | 63.0 | 52.1 | 56.2 | 62.5 | 61.9 |
| Retention rate of elongation at break after aging under 100°C×48h /% | 47.2 | 46.3 | 45.2 | 44.9 | 43.5 | 43.0 | 32.3 | 36.1 | 41.5 | 41 |

As can be seen from Table 2, the mechanical properties before aging of the rubber composition in Examples 1-2 containing the compound of formula I, Examples 3-4 containing the compound of formula II, and Examples 5-6 containing the compound of formula III are close to those of the rubber composition in Comparative Examples 3-4 containing antidegradant 6PPD. The retention rate of tensile strength and the retention rate of elongation at break after aging of the rubber composition of Comparative Example 1 are lower than those of Comparative Example 2, indicating that the addition of cobalt salt is not conducive to thermal oxidative aging performance. Compared to the rubber composition of Comparative Example 1, the retention rate of tensile strength and the retention rate of elongation at break after aging of the rubber composition in Examples 1-2 containing Compound I, Examples 3-4 containing Compound II, and Examples 5-6 containing Compound III are significantly improved, indicating that Compound I, Compound II, and Compound III can provide excellent thermal oxidative aging resistance and protection against metal ions for a rubber composition. Especially at the same dosage of an antidegradant, the retention rate of tensile strength and the retention rate of elongation at break after thermal oxidative aging in the presence of metal ion for the rubber composition in Examples 1-2 containing Compound I, Examples 3-4 containing Compound II, and Examples 5-6 containing Compound III are higher than those of the rubber composition in Comparative Examples 3-4 containing antidegradant 6PPD, indicating that Compound I, Compound II, and Compound III have better effects in improving the thermal oxidative aging resistance and protection against metal ion of a rubber composition compared to antidegradant 6PPD.

Test Example 2: Adhesion Performance to steel wire

An adhesion strength to steel cord before and after thermal oxidative aging for the vulcanized rubber of Examples 1-6 and Comparative Examples 1-4 is tested according to the following standards, and test results are shown in Table 3:
Adhesion strength: GB/T 16586-1996, Determination of adhesion strength between vulcanized rubber and steel cord.
Thermal oxidative aging test: GB/T 3512-2014, Vulcanized or thermoplastic rubber, Hot-air-accelerated aging and heat resistance tests, Aging condition of 100°C × 96h.

Retention rate of adhesion strength after aging = (Adhesion strength after aging / Adhesion strength before aging) × 100%

Table 3: Adhesion strength to steel cord before and after aging for rubber composition of Examples 1-6 and Comparative Examples 1-4

| | Ex.1 | Ex.2 | Ex.3 | Ex.4 | Ex.5 | Ex.6 | Com. Ex.1 | Com. Ex.2 | Com. Ex.3 | Com. Ex.4 |
|---|---|---|---|---|---|---|---|---|---|---|
| Adhesion strength before aging/(N/mm) | 77 | 76 | 76 | 76 | 75 | 74 | 72 | 68 | 74 | 74 |
| Retention rate of adhesion strength after aging under 100°C×96h /% | 92.7 | 91.4 | 89.4 | 88.7 | 86.1 | 85.5 | 78.5 | 77.6 | 85.3 | 84.5 |

As can be seen from Table 3, compared to the vulcanized rubber of Comparative Example 2 without cobalt salt, the vulcanized rubber of Comparative Example 1 with cobalt salt has higher adhesion strength, indicating that the addition of cobalt salt improves rubber-steel wire adhesion performance. The adhesion strength of the vulcanized rubber in Examples 1-2 containing Compound I and Examples 3-4 containing Compound II is higher than that of the vulcanized

rubber in Comparative Example 1 without antidegradant and Comparative Examples 3-4 containing antidegradant 6PPD, indicating that Compound I and Compound II can improve rubber-steel wire adhesion performance. The retention rates of adhesion strength after thermal oxidative aging of the vulcanized rubber in Examples 1-2 containing Compound I, Examples 3-4 containing Compound II, and Examples 5-6 containing Compound III are higher than those of the vulcanized rubber in Comparative Examples 3-4 containing antidegradant 6PPD, indicating that Compound I, Compound II, and Compound III can improve the rubber-steel wire adhesion performance after thermal oxidative aging in the presence of metal ion.

## Claims

1. A rubber composition, wherein raw materials of the rubber composition comprise 100 parts by mass of a diene elastomer, 0.2-5 parts by mass of a cobalt salt, and 0.5-5 parts by mass of a compound of formula A:

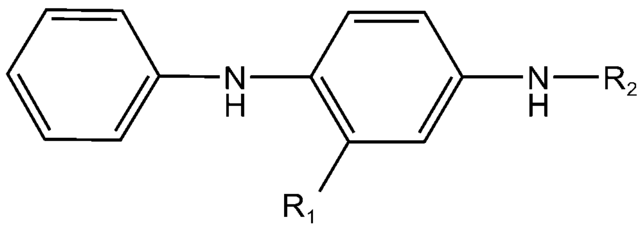

(A)

wherein in formula A, $R_1$ is selected from C1-C8 alkyl, and $R_2$ is selected from C1-C8 alkyl.

2. The rubber composition according to claim 1, wherein in formula A, $R_1$ is methyl, and $R_2$ is selected from C3-C6 alkyl.

3. The rubber composition according to claim 1, wherein the compound of formula A is a compound of formula I, a compound of formula II, or a compound of formula III:

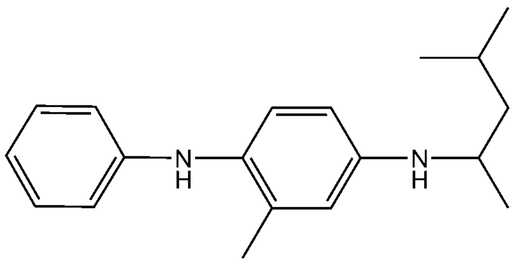

(I) ;

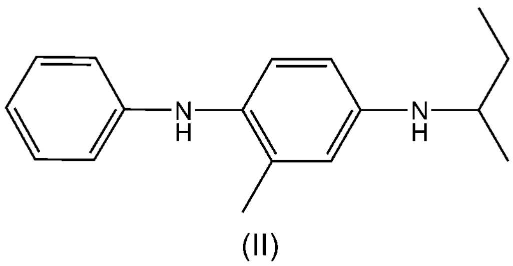

(II) ;

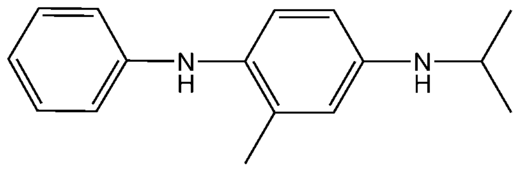

(III) .

4. The rubber composition according to claim 1, wherein the diene elastomer is natural rubber.

5. The rubber composition according to claim 1, wherein the raw materials of the rubber composition further comprise 2-10 parts by mass of sulfur.

6. The rubber composition according to claim 1, wherein in the raw materials of the rubber composition, an amount of a compound of formula A is 1-3 parts by mass.

7. The rubber composition according to claim 1, wherein the rubber composition has one or more of the following characteristics:

the raw materials of the rubber composition further comprise 30-70 parts by mass of a reinforcing filler; the reinforcing filler preferably comprises carbon black and silica;
the raw materials of the rubber composition further comprise 2-15 parts by mass of an activator; preferably, the activator is zinc oxide;
the raw materials of the rubber composition further comprise 0.5-5 parts by mass of a tackifying resin;
the raw materials of the rubber composition further comprise 1-10 parts by mass of a rubber adhesive;
the raw materials of the rubber composition further comprise 0.5-5 parts by mass of an accelerator; preferably, the accelerator is N,N'-dicyclohexyl-2-benzothiazolesulfenamide;
the raw materials of the rubber composition further comprise 0.02-0.5 parts by mass of a rubber scorch retarder.

8. A rubber product, wherein the rubber product comprises the rubber composition according to any one of claims 1-7; preferably, the rubber product is a tire belt.

9. Use of a compound of formula A in improving mechanical performance and adhesion performance to steel wire after thermal oxidative aging in the presence of metal ion for a rubber composition:

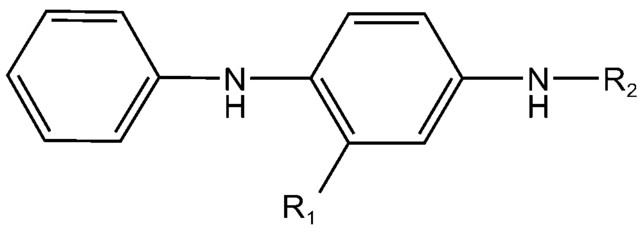

(A)

wherein in formula A, $R_1$ is selected from C1-C8 alkyl, and $R_2$ is selected from C1-C8 alkyl;
preferably, in formula A, $R_1$ is methyl, and $R_2$ is selected from C3-C6 alkyl;
preferably, the compound of formula A is a compound of formula I, a compound of formula II, or a compound of formula III:

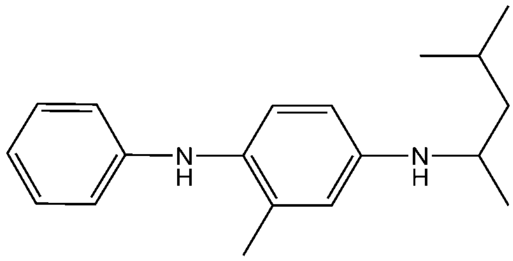

(I) ;

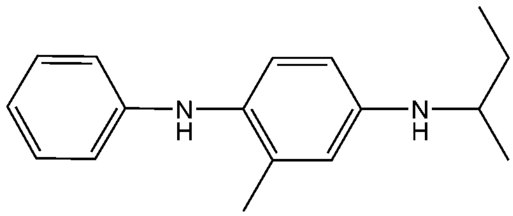

(II) ;

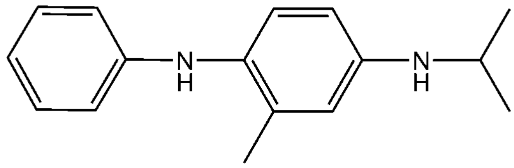

(III) .

**10.** The use according to claim 9, wherein the use comprises adding 0.5-5 parts by mass of the compound of formula A to the raw materials of a rubber composition which comprises 100 parts by mass of a diene elastomer and 0.2-5 parts by mass of a cobalt salt.

## INTERNATIONAL SEARCH REPORT

International application No. **PCT/CN2024/094380**

**A. CLASSIFICATION OF SUBJECT MATTER**

C07C213/00(2006.01)i; C07C209/00(2006.01)i; C07C211/54(2006.01)i; C07C209/00(2006.01)i; C08K5/18(2006.01)i; C08L7/00(2006.01)i; C08L9/00(2006.01)i

According to International Patent Classification (IPC) or to both national classification and IPC

**B. FIELDS SEARCHED**

Minimum documentation searched (classification system followed by classification symbols)

IPC: C07C, C08K, C08L, C07C213, C07C209, C07C211, C07C209, C08K5, C08L7, C08L9

Documentation searched other than minimum documentation to the extent that such documents are included in the fields searched

Electronic data base consulted during the international search (name of data base and, where practicable, search terms used)

CNTXT, CNABS, CNKI, ENTXT, DWPI, ENTXTC, STN: 橡胶, 二烯, 抗氧化剂, 抗氧剂, 对苯二胺, 钴, 权利要求1中结构式A, rubber, diene, antioxidant, phenylenediamine, cobalt

**C. DOCUMENTS CONSIDERED TO BE RELEVANT**

| Category* | Citation of document, with indication, where appropriate, of the relevant passages | Relevant to claim No. |
|---|---|---|
| Y | CN 105860162 A (QINGDAO DOUBLESTAR TIRE INDUSTRIAL CO., LTD.) 17 August 2016 (2016-08-17)<br>description, paragraphs [0004]-[0011] | 1-8 |
| Y | CN 101370766 A (CHEMTURA CORP.) 18 February 2009 (2009-02-18)<br>description, lines 3-11, and page 8, paragraph 2 | 1-8 |
| A | CN 106700140 A (BEIJING TONGCHENG CHUANGZHAN TECHNOLOGY CO., LTD.) 24 May 2017 (2017-05-24)<br>entire document | 1-10 |
| A | CN 113072741 A (EVE RUBBER INSTITUTE CO., LTD.) 06 July 2021 (2021-07-06)<br>entire document | 1-10 |
| A | JP 2010254752 A (BRIDGESTONE CORP.) 11 November 2010 (2010-11-11)<br>entire document | 1-10 |
| A | WO 2018102289 A1 (COMPAGNIE GENERALE DES ETABLISSEMENTS MICHELIN et al.) 07 June 2018 (2018-06-07)<br>entire document | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 August 2024** | **09 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/094380**

| Patent document cited in search report | | | Publication date (day/month/year) | Patent family member(s) | | | Publication date (day/month/year) |
|---|---|---|---|---|---|---|---|
| CN | 105860162 | A | 17 August 2016 | None | | | |
| CN | 101370766 | A | 18 February 2009 | ATE | 510814 | T1 | 15 June 2011 |
| | | | | WO | 2007087195 | A1 | 02 August 2007 |
| | | | | US | 2007173668 | A1 | 26 July 2007 |
| | | | | US | 7361788 | B2 | 22 April 2008 |
| | | | | EP | 1976821 | A1 | 08 October 2008 |
| | | | | EP | 1976821 | B1 | 25 May 2011 |
| | | | | JP | 2009524660 | A | 02 July 2009 |
| | | | | JP | 4950224 | B2 | 13 June 2012 |
| | | | | RU | 2008134529 | A | 27 February 2010 |
| | | | | RU | 2421444 | C2 | 20 June 2011 |
| CN | 106700140 | A | 24 May 2017 | CN | 106700140 | B | 13 September 2019 |
| CN | 113072741 | A | 06 July 2021 | CN | 113072741 | B | 15 July 2022 |
| JP | 2010254752 | A | 11 November 2010 | None | | | |
| WO | 2018102289 | A1 | 07 June 2018 | WO | 2018101916 | A1 | 07 June 2018 |

Form PCT/ISA/210 (patent family annex) (July 2022)